(19) Europäisches Patentamt
European Patent Office
Office européen des brevets

(11) Veröffentlichungsnummer: **0 081 047**
**B1**

(12) # EUROPÄISCHE PATENTSCHRIFT

(45) Veröffentlichungstag der Patentschrift: **18.12.85**

(21) Anmeldenummer: **82108875.4**

(22) Anmeldetag: **25.09.82**

(51) Int. Cl.⁴: **C 07 D 491/048**, C 07 D 233/22, C 07 J 43/00 // (C07D491/048, 307:00, 235:00)

(54) **Verfahren zur Herstellung eines optisch aktiven Lactons und neue Ausgangsprodukte in diesem Verfahren.**

(30) Priorität: **07.12.81 CH 7803/81**
**18.08.82 CH 4940/82**

(43) Veröffentlichungstag der Anmeldung:
**15.06.83 Patentblatt 83/24**

(45) Bekanntmachung des Hinweises auf die Patenterteilung:
**18.12.85 Patentblatt 85/51**

(84) Benannte Vertragsstaaten:
**AT BE CH DE FR GB IT LI NL**

(56) Entgegenhaltungen:
**DE - A - 2 058 248**

(73) Patentinhaber: **F. HOFFMANN-LA ROCHE & CO. Aktiengesellschaft, CH-4002 Basel (CH)**

(72) Erfinder: **Pauling, Horst, Dr., Ruchholzstrasse 39, CH-4103 Bottmingen (CH)**
Erfinder: **Wehrli, Christof, Rheinstrasse 40, CH-4127 Birsfelden (CH)**
Erfinder: **Vorsanger, Jean-Jacques, Dr., rue Mayeur Debras 1, B-4296 Hannut (BE)**

(74) Vertreter: **Cottong, Norbert A. et al, Grenzacherstrasse 124 Postfach 3255, CH-4002 Basel (CH)**

EP 0 081 047 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung des optisch aktiven Lactons der Formel

(3aS, (6aR)

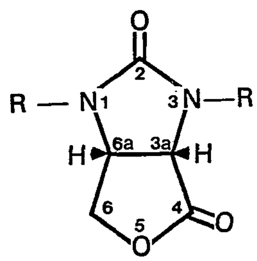

I

worin R den Benzylrest darstellt.

Dieses optisch aktive Lacton der Formel I ist ein bekanntes, wertvolles Zwischenprodukt in der Synthese von (+)-Biotin, sowie von Derivaten und verwandten Verbindungen hiervon.

Unter der Bezeichnung «(3aS,6aR)» im Zusammenhang mit der Formel I ist im Rahmen der vorliegenden Erfindung derjenige Antipode zu verstehen, welcher in Benzol oder Chloroform rechtsdrehend ist. Dieser Antipode wird im nachfolgenden als (+)-Lacton bezeichnet.

Aus der deutschen Patentschrift Nr. 2 058 248 ist bereits ein Verfahren zur Herstellung des (+)-Lactons der Formel I bekannt. Hierbei werden die racemischen Halbester der Formel

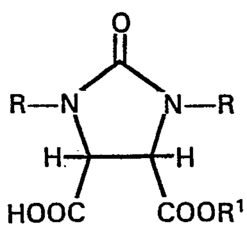

A

worin R die obige Bedeutung hat und $R^1$ den Cholesteryl- oder den Cyclohexylrest bedeutet,
in ihre optischen Antipoden aufgetrennt und der gewünschte Antipode wird in das (+)-Lacton der Formel I übergeführt. Dieses Verfahren hat jedoch den Nachteil, dass der bei der Racematspaltung anfallende unerwünschte Antipode recyclisiert werden muss.

Es bestand somit ein Bedürfnis nach einem Verfahren, gemäss welchem auch dieser unerwünschte Antipode in das gewünschte (+)-Lacton der Formel I übergeführt werden kann. Dies ist erfindungsgemäss nunmehr gelungen. Es wurde nämlich überraschend gefunden, dass dieser unerwünschte Antipode nach Überführung in das entsprechende Säurechlorid selektiv mit einem Dialkylaluminiumhydrid oder einem komplexen Borhydrid zum (+)-Lacton der Formel I reduziert werden kann.

Unter «Dialkylaluminiumhydriden» sind im Rahmen der vorliegenden Erfindung Aluminiumhydride zu verstehen, deren Alkylreste 2-8 Kohlenstoffatome aufweisen und geradkettig oder verzweigt sein können. Als Beispiele können hier genannt werden Diäthylaluminiumhydrid, Diisoputylaluminiumhydrid (DIBAH), Di-n-hexylaluminiumhydrid und dergleichen.

Unter «komplexen Borhydriden» sind im Rahmen der vorliegenden Erfindung insbesondere solche zu verstehen, worin das Kation ein Alkalimetall, wie Lithium, Natrium oder Kalium, oder ein Tetraalkylammoniumion, wie Tetrabutylammonium, sein kann. Ferner sind hier solche Hydride zu verstehen in welchen ein Wasserstoffatom durch die Cyanogruppe ersetzt ist, wie z.B. Lithium-, Natrium- oder Kaliumcyanoborhydrid.

Das erfindungsgemässe Verfahren ist demnach dadurch gekennzeichnet, dass man eine optisch aktive Verbindung der allgemeinen Formel

(4S, 5R)

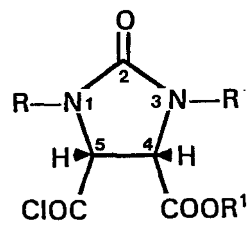

II

worin R und $R^1$ die obige Bedeutung haben,
mit einem Dialkylaluminiumhydrid oder einem komplexen Borhydrid reduziert.

Bei der Spaltung der racemischen Verbindungen der Formel A fallen die unerwünschten Antipoden in Form von Salzen mit den als Spaltungsmitteln verwendeten Basen an, aus welchen dann leicht in an sich bekannter Weise die entsprechenden optisch aktiven Säuren freigesetzt werden können.

Diese Säuren können in an sich bekannter Weise in die optisch aktiven Säurechloride der Formel II übergeführt werden. Zweckmässig erfolgt diese Überführung mittels eines geeigneten Halogenierungsmittels in einem inerten organischen Lösungsmittel. Als Halogenierungsmittel können insbesondere genannt werden Thionylchlorid, Phosphortrichlorid, Phorphorpentachlorid und dergleichen. Besonders bevorzugt ist das Thionylchlorid. Als inerte organische Lösungsmittel können genannt werden aromatische und aliphatische Kohlenwasserstoffe, wie z.B. Benzol, Toluol, Hexan, Isooctan und dergleichen.

Bevorzugte Lösungsmittel sind im vorliegenden Fall die aromatischen Kohlenwasserstoffe und insbesondere Benzol und Toluol. Die Temperatur und der Druck sind keine kritischen Grössen in dieser Reaktion, welche somit ohne weiteres bei Normaldruck und bei Temperaturen von etwa Raumtemperatur bis etwa 60°C durchgeführt werden kann.

Die optisch aktiven Säurechloride der Formel II sind neue Verbindungen und als solche ebenfalls Gegenstand der vorliegenden Erfindung.

Die Reduktion einer Verbindung der Formel II erfolgt erfindungsgemäss mit einem Dialkylaluminiumhydrid oder einem komplexen Borhydrid, wobei Lithiumborhydrid und Natriumborhydrid bevorzugt sind.

Die Reaktion erfolgt zweckmässig in einem gegenüber Säurechloriden unter den bevorzugten Reaktionsbedingungen praktisch inerten Lösungsmittel, in welchem zudem die verwendeten Hydride zumindest teilweise löslich sind. Zweckmässige und allgemein bekannte Lösungsmittel sind in diesem Fall die üblicherweise im Zusammenhang mit den entsprechenden Hydriden verwendbaren Lösungsmittel, wie z.B. aliphatische Polyäther, wie Mono- und Diglyme, sowie Tetrahydrofuran und Dimethylformamid, Toluol oder Gemische bzw. wässrige Gemische hiervon.

Damit die gewünschte Reduktion nicht zu langsam abläuft und dabei unerwünschte Nebenprodukte gebildet werden, werden die erwähnten Hydride vorzugsweise in gelöster Form zum Reaktionsgemisch gegeben.

Als Lösungsmittel für in wässrigem Medium anwendbare Hydride, wie z.B. das Natriumborhydrid, eignen sich im vorliegenden Fall neben Dimethylformamid auch noch Diglyme und insbesondere Wasser. Die Temperatur während der Reduktion beträgt in diesem Fall zweckmässig von etwa 40°C bis etwa —30°C, vorzugsweise von etwa 0°C bis etwa —30°C und insbesondere von etwa —10°C bis etwa —20°C.

Als Lösungsmittel für wasser empfindliche Hydride, wie z.B. Lithiumborhydrid oder auch Diisobutylaluminiumhydrid, eignen sich im vorliegenden Fall insbesondere Kohlenwasserstoffe wie Toluol und dergleichen oder auch Äther wie Tetrahydrofuran. Die Temperatur während der Reduktion beträgt in diesem Fall zweckmässig von etwa Raumtemperatur bis etwa —20°C, vorzugsweise von etwa 10°C bis etwa —10°C und insbesondere von etwa 5°C bis etwa —5°C.

Der Druck ist hierbei keine kritische Grösse und die Reaktion kann in geeigneter Weise bei Normaldruck durchgeführt werden. Die Menge an verwendetem Hydrid liegt bei der erfindungsgemässen Reduktion zweckmässig zwischen etwa 0,5 und etwa 2,5 Mol pro Mol eingesetztes Säurechlorid. So liegt die bevorzugte Menge bei Verwendung von Natriumborhydrid bei etwa 1 bis etwa 1,25 Mol und bei Verwendung von Lithiumborhydrid bei etwa 0,8 bis etwa 0,85 Mol und bei DIBAH bei etwa 2 Mol pro Mol Säurechlorid.

Da bei Verwendung von wässrigen Systemen als Konkurrenzreaktion zur Reduktion auch die Hydrolyse der Säurechloridfunktion auftreten kann, ist es zweckmässig, die in wässrigem Medium anwendbaren Hydride rasch und bei tiefer Temperatur zur Lösung der Verbindung der Formel II zu geben.

Eine besonders bevorzugte Ausführungsform des erfindungsgemässen Verfahrens besteht z.B. darin, dass man eine auf ca. —20°C gekühlte Lösung einer Verbindung der Formel II in Tetrahydrofuran mit einer wässrigen Natriumborhydridlösung reduziert.

Eine weitere bevorzugte Ausführungsform besteht darin, dass man eine auf ca. 0°C gekühlte Lösung einer Verbindung der Formel II in Tetrahydrofuran oder in Toluol mit einer Lösung von Lithiumborhydrid in Tetrahydrofuran reduziert.

*Beispiel 1*

4,55 g (10 mMol) Cyclohexyl (4S,5R)-1,3-dibenzyl-5-chlorcarbonyl-2-oxo-4-imidazolidincarboxylat werden in 10 ml Tetrahydrofuran gelöst und mit einem Trockeneis/Aceton-Bad auf —20°C abgekühlt. Unter intensivem Rühren wird dann eine Lösung von 0,47 g (12,5 mMol) Natriumborhydrid in 3 ml Wasser mit einer solchen Geschwindigkeit zugetropft, dass die Reaktionstemperatur bei —20°C gehalten wird (Zutropfzeit ca. 3 Minuten). Hierauf wird das Kühlbad entfernt und die Reaktionsmischung noch 10 Minuten gerührt. Die Temperatur steigt dabei von —20°C auf etwa 0°C an. Zu der Reaktionsmischung werden bei 0°C bis —10°C (Eisbadkühlung) eine Lösung von 8 ml 25%iger Salzsäure und 30 ml Wasser innert 5 Minuten zugetropft. Am Anfang tritt starkes Schäumen auf. Das Reaktionsgemisch wird dann 20 Minuten bei 60°C gerührt und anschliessend im Wasserstrahlvakuum auf ca. 15 ml eingeengt. Dazu werden dann 15 ml Wasser gegeben und eine Stunde bei 0°C stehen gelassen. Das auskristallisierte Rohprodukt wird abgenutscht, mit Wasser nachgewaschen und im Vakuum 4 Stunden bei 60°C getrocknet. Das Kristallisat wird in 15 ml Isopropanol heiss gelöst und 18 Stunden bei 0°C kristallisieren gelassen. Das Produkt wird dann abgenutscht und zweimal mit 5 ml eiskaltem Isopropanol nachgewaschen. Nach 6 Stunden Trocknen bei 60°C im Vakuum wird ein erstes Kristallisat erhalten: 2,84 g (88%) mit einem Schmelzpunkt von 116° bis 117°C.

Aus der Mutterlauge wird durch Kristallisieren aus 3 ml Isopropanol und zweimaligem Nachwaschen mit 1 ml Isopropanol ein zweites Kristallisat erhalten: 0,30 g (9,3%) mit einem Schmelzpunkt von 116° bis 117°C.

Die Gesamtausbeute beträgt somit 3,14 g (97%) (3aR,6aR)-1,3-Dibenzyldihydro-1H-furo[3,4-d]-imidazol-2,4(3H,3aH)-dion. Schmelzpunkt = 116° bis 117°C; $[\alpha]_D^{20}$ + 60,6° (1% in $CHCl_3$).

Das als Ausgangsmaterial verwendete Cyclohexyl (4S,5R)-1,3-Dibenzyl-5-chlorcarbonyl-2-oxo-4--imidazolidincarboxylat kann wie folgt hergestellt werden:

In einem 100 ml Sulfierkolben werden unter einer Argonatmosphäre 4,365 g (10 mMol) 4-Cyclohexyl--5-hydrogen (4S,5R)-1,3-dibenzyl-2-oxo-4,5-imidazolidindicarboxylat sowie 10 ml Toluol und 1,5 ml (20 mMol) Thionylchlorid vereinigt und 2 Stunden bei 40°C gerührt (die Gasentwicklung ist nach etwa einer Stunde beendet). Das Toluol und das überschüssige Thionylchlorid werden im Wasserstrahlvakuum mit vorgeschalteter $CO_2$-Kühlfalle abdestilliert (Ölbad 55°C). Wenn die Destillation beendet ist, wird auf Raumtemperatur abgekühlt und das Vakuum mit Argon aufgehoben. Man erhält so 4,55 g Cyclohexyl (4S,5R)-1,3-dibenzyl-5-chlorcarbonyl-2--oxo-4-imidazolidincarboxylat.

*Beispiel 2*

45,5 g (100 mMol) Cyclohexyl (4S,5R)-1,3-dibenzyl-5-chlorcarbonyl-2-oxo-4-imidazolidincarboxylat (hergestellt gemäss Beispiel 1) werden in 50 ml Tetrahydrofuran gelöst. Zu der klaren Lösung werden bei 0°C innert 60 Minuten 80 ml einer 1M Lithium-

borhydrid-Lösung in Tetrahydrofuran (80 mMol) zugetropft. Die Reaktionsmischung wird dann noch 30 Minuten bei 0°C gerührt. Dann werden bei 0°C 100 ml 1N Salzsäure vorsichtig zugetropft. Das Reaktionsgemisch wird dann 30 Minuten bei 70°C gerührt und anschliessend im Rotationsverdampfer auf 100 ml eingeengt. Dazu werden 200 ml Wasser gegeben und die Mischung 18 Stunden bei 0°C kristallisieren gelassen. Das auskristallisierte Rohprodukt wird abgenutscht und mit Wasser neutral gewaschen. Nach 4 Stunden Trocknen im Vakuum bei 60°C wird das Rohprodukt in 100 ml Isopropanol heiss gelöst und dann 18 Stunden bei 0°C kristallisieren gelassen. Das Produkt wird dann abgenutscht und zweimal mit je 20 ml eiskaltem Isopropanol nachgewaschen. Nach 6 Stunden Trocknen bei 60°C im Wasserstrahlvakuum erhält man 26,5 g (3aR,6aR)-1,3-Dibenzyldihydro-1H-furo[3,4-d]-imidazol-2,4(3H,3aH)-dion mit einem Schmelzpunkt von 116° bis 117°C.

Aus der Mutterlauge werden durch Kristallisieren noch weitere 2,35 g Produkt mit einem Schmelzpunkt von 116° bis 117°C erhalten, was einer Gesamtausbeute von 28,85 g oder 89,5% der Theorie entspricht.

**Patentansprüche für die Vertragsstaaten:**
BE, CH, DE, FR, GB, IT, LI, NL

1. Verfahren zur Herstellung des optisch aktiven Lactons der Formel

(3aS, (6aR)

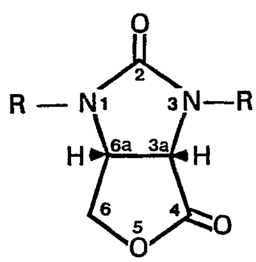

I

worin R den Benzylrest darstellt,
dadurch gekennzeichnet, dass man eine optisch aktive Verbindung der allgemeinen Formel

(4S, 5R)

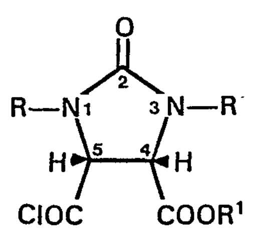

II

worin R die obige Bedeutung hat und $R^1$ den Cholesteryl- oder den Cyclohexylrest bedeutet,
mit einem Dialkylaluminiumhydrid oder einem komplexen Borhydrid reduziert.

2. Verfahren nach Anspruch 1, dadurch gekennzeichnet, dass man die Reduktion mittels Natriumborhydrid oder Lithiumborhydrid durchführt.

3. Verfahren nach Anspruch 1 oder 2, dadurch gekennzeichnet, dass man eine Verbindung der Formel II in Tetrahydrofuran gelöst mit einer wässrigen Natriumborhydridlösung reduziert.

4. Verfahren nach Anspruch 1 oder 2, dadurch gekennzeichnet, dass man eine Verbindung der Formel II in Tetrahydrofuran oder Toluol gelöst mit einer Lösung von Lithiumborhydrid in Tetrahydrofuran reduziert.

5. Verfahren nach einem der Ansprüche 1 bis 3, dadurch gekennzeichnet, dass man die Reduktion bei einer Temperatur von etwa 40°C bis etwa —30°C, vorzugsweise bei etwa 0°C bis etwa —30°C, insbesondere bei etwa —10°C bis etwa —20°C durchführt.

6. Verfahren nach einem der Ansprüche 1, 2 oder 4, dadurch gekennzeichnet, dass man die Reduktion bei einer Temperatur von etwa Raumtemperatur bis etwa —20°C, vorzugsweise bei etwa 10°C bis etwa —10°C, insbesondere bei etwa 5°C bis etwa —5°C durchführt.

7. Verbindungen der allgemeinen Formel

(4S, 5R)

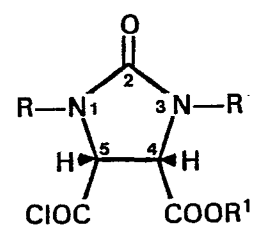

II

worin R und $R_1$ die in Anspruch 1 angegebene Bedeutung haben.

**Patentansprüche für den Vertragsstaat:** AT

1. Verfahren zur Herstellung des optisch aktiven Lactons der Formel

(3aS, (6aR)

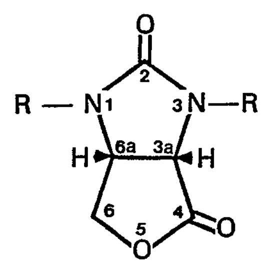

I

worin R den Benzylrest darstellt,
dadurch gekennzeichnet, dass man eine optisch aktive Verbindung der allgemeinen Formel

(4S, 5R)

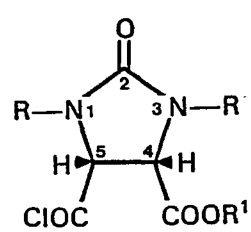

II

worin R die obige Bedeutung hat und $R^1$ den Cholesteryl- oder den Cyclohexylrest bedeutet,
mit einem Dialkylaluminiumhydrid oder einem komplexen Borhydrid reduziert.

2. Verfahren nach Anspruch 1, dadurch gekennzeichnet, dass man die Reduktion mittels Natriumborhydrid oder Lithiumborhydrid durchführt.

3. Verfahren nach Anspruch 1 oder 2, dadurch gekennzeichnet, dass man eine Verbindung der Formel II in Tetrahydrofuran gelöst mit einer wässrigen Natriumborhydridlösung reduziert.

4. Verfahren nach Anspruch 1 oder 2, dadurch gekennzeichnet, dass man eine Verbindung der Formel II in Tetrahydrofuran oder Toluol gelöst mit einer Lösung von Lithiumborhydrid in Tetrahydrofuran reduziert.

5. Verfahren nach einem der Ansprüche 1 bis 3, dadurch gekennzeichnet, dass man die Reduktion bei einer Temperatur von etwa 40°C bis etwa —30°C, vorzugsweise bei etwa 0°C bis etwa —30°C, insbesondere bei etwa —10°C bis etwa —20°C durchführt.

6. Verfahren nach einem der Ansprüche 1, 2 oder 4, dadurch gekennzeichnet, dass man die Reduktion bei einer Temperatur von etwa Raumtemperatur bis etwa —20°C, vorzugsweise bei etwa 10°C bis etwa —10°C, insbesondere bei etwa 5°C bis etwa —5°C durchführt.

**Claims for the Contracting States:**
BE, CH, DE, FR, GB, IT, LI, NL

1. A process for the manufacture of the optically active lactone of the formula

(3aS, (6aR)

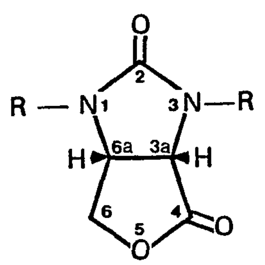

I

wherein R represents the benzyl residue,
characterized by reducing an optically active compound of general formula

(4S, 5R)

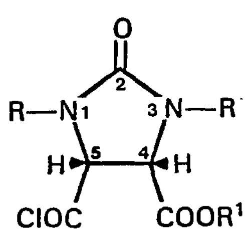

II

wherein R has the above significance and $R^1$ signifies the cholesteryl residue or the cyclohexyl residue, with a dialkylaluminium hydride or a complex borohydride.

2. A process according to claim 1, characterized in that the reduction is carried out by means of sodium borohydride or lithium borohydride.

3. A process according to claim 1 or 2, characterized in that a compound of formula II dissolved in tetrahydrofuran is reduced with an aqueous sodium borohydride solution.

4. A process according to claim 1 or 2, characterized in that a compound of formula II dissolved in tetrahydrofuran or toluene is reduced with a solution of lithium borohydride in tetrahydrofuran.

5. A process according to any one of claims 1 to 3, characterized in that the reduction is carried out at a temperature of about 40°C to about —30°C, preferably at about 0°C to about —30°C, especially at about —10°C to about —20°C.

6. A process according to any one of claims 1, 2 or 4, characterized in that the reduction is carried out at a temperature of about room temperature to about —20°C, preferably at about 10°C to about —10°C, especially at about 5°C to about —5°C.

7. Compounds of the general formula

(4S, 5R)

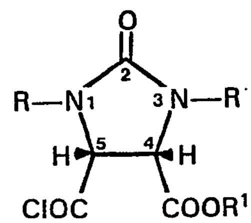

II

wherein R and $R^1$ have the significance given in claim 1.

**Claims for the Contracting State:** AT

1. A process for the manufacture of the optically active lactone of the formula

(3aS, (6aR)

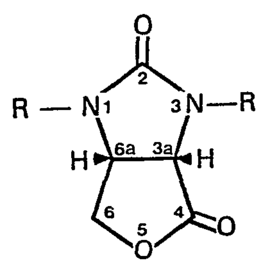

I

wherein R represents the benzyl residue,
characterized by reducing an optically active compound of general formula

(4S, 5R)

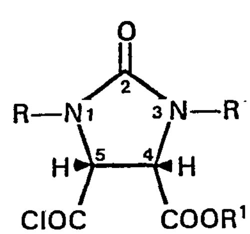

II

wherein R has the above significance and $R^1$ signifies the cholesteryl residue or the cyclohexyl residue, with a dialkylaluminium hydride or a complex borohydride.

2. A process according to claim 1, characterized in that the reduction is carried out by means of sodium borohydride or lithium borohydride.

3. A process according to claim 1 or 2, characterized in that a compound of formula II dissolved in tetrahydrofuran is reduced with an aqueous sodium borohydride solution.

4. A process according to claim 1 or 2, characterized in that a compound of formula II dissolved in tetrahydrofuran or toluene is reduced with a solution of lithium borohydride in tetrahydrofuran.

5. A process according to any one of claims 1 to 3, characterized in that the reduction is carried out at a temperature of about 40°C to about —30°C, preferably at about 0°C to about —30°C, especially at about —10°C to about —20°C.

6. A process according to any one of claims 1, 2 or 4, characterized in that the reduction is carried out at a temperature of about room temperature to about —20°C, preferably at about 10°C to about —10°C, especially at about 5°C to about —5°C.

**Revendications pour les Etats contractants:**
BE, CH, DE, FR, GB, IT, LI, NL

1. Procédé de préparation de la lactone optiquement active de formule

(3aS, (6aR) 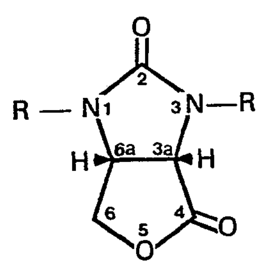 I

dans laquelle R représente le reste benzyle, caractérisé en ce que l'on réduit un composé optiquement activ de formule générale

(4S, 5R) 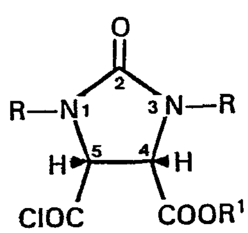 II

dans laquelle R a la signification indiquée ci-dessus et $R^1$ représente un reste cholestéryle ou cyclohexyle, à l'aide d'un hydrure de dialkyl-aluminium ou d'un borohydrure complexe.

2. Procédé selon la revendication 1, caractérisé en ce que l'on réduit à l'aide du borohydrure de sodium ou du borohydrure de lithium.

3. Procédé selon la revendication 1 ou 2, caractérisé en ce que l'on dissout un composé de formule II dans le tétrahydrofuranne et on réduit à l'aide d'une solution aqueuse de borohydrure de sodium.

4. Procédé selon la revendication 1 ou 2, caractérisé en ce que l'on dissout un composé de formule II dans le tétrahydrofuranne ou le toluène et on réduit à l'aide d'une solution de borohydrure de lithium dans le tétrahydrofuranne.

6. Procédé selon l'une des revendications 1 à 3, caractérisé en ce que l'on réduit à une température d'environ 40°C à environ —30°C, de préférence d'environ 0°C à environ —30°C, plus spécialement d'environ —10°C à environ —20°C.

6. Procédé selon l'une des revendications 1, 2 ou 4, caractérisé en ce que l'on réduit à une température allant du voisinage de la température ambiante jusqu'à —20°C environ, de préférence à une température d'environ 10°C à environ —10°C, plus particulièrement d'environ 5°C à environ —5°C.

7. Composé de formule générale

(4S, 5R) 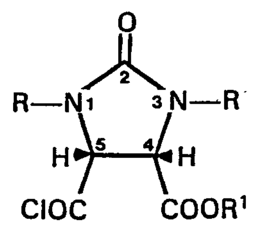 II

dans laquelle R et $R^1$ ont les significations indiquées dans la revendication 1.

**Revendications pour l'Etat contractant:** AT

1. Procédé de préparation de la lactone optiquement active de formule

(3aS, (6aR) 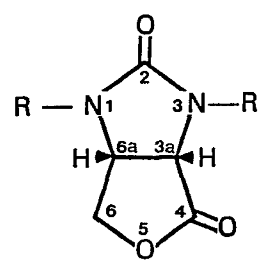 I

dans laquelle R représente le reste benzyle, caractérisé en ce que l'on réduit un composé optiquement activ de formule générale

(4S, 5R) 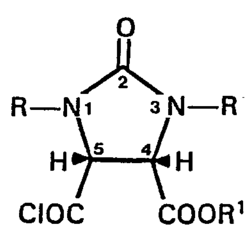 II

dans laquelle R a la signification indiquée ci-dessus et $R^1$ représente un reste cholestéryle ou cyclohexyle, à l'aide d'un hydrure de dialkyl-aluminium ou d'un borohydrure complexe.

2. Procédé selon la revendication 1, caractérisé en ce que l'on réduit à l'aide du borohydrure de sodium ou du borohydrure de lithium.

3. Procédé selon la revendication 1 ou 2, caractérisé en ce que l'on dissout un composé de formule II dans le tétrahydrofuranne et on réduit à l'aide d'une solution aqueuse de borohydrure de sodium.

4. Procédé selon la revendication 1 ou 2, caractérisé en ce que l'on dissout un composé de formule II dans le tétrahydrofuranne ou le toluène et on réduit à l'aide d'une solution de borohydrure de lithium dans le tétrahydrofuranne.

6. Procédé selon l'une des revendications 1 à 3, caractérisé en ce que l'on réduit à une température d'environ 40°C à environ —30°C, de préférence d'environ 0°C à environ —30°C, plus spécialement d'environ —10°C à environ —20°C.

6. Procédé selon l'une des revendications 1, 2 ou 4, caractérisé en ce que l'on réduit à une température allant du voisinage de la température ambiante jusqu'à —20°C environ, de préférence à une température d'environ 10°C à environ —10°C, plus particulièrement d'environ 5°C à environ —5°C.